(19) Europäisches Patentamt
European Patent Office
Office européen des brevets

(11) Veröffentlichungsnummer: **0 179 223**
**B1**

(12) **EUROPÄISCHE PATENTSCHRIFT**

(45) Veröffentlichungstag der Patentschrift: **26.07.89**

(51) Int. Cl.⁴: **C 07 C 179/10**, C 07 C 178/00, C 11 D 3/39, D 06 L 3/02

(21) Anmeldenummer: **85110507.2**

(22) Anmeldetag: **21.08.85**

(54) **Verfahren zur Herstellung stabilisierter substituierter Diperoxybernsteinsäuren.**

(30) Priorität: **20.10.84 DE 3438529**

(43) Veröffentlichungstag der Anmeldung: **30.04.86 Patentblatt 86/18**

(45) Bekanntmachung des Hinweises auf die Patenterteilung: **26.07.89 Patentblatt 89/30**

(84) Benannte Vertragsstaaten: **AT BE CH DE FR GB IT LI LU NL SE**

(56) Entgegenhaltungen:
**EP-A- 0 083 560**
**EP-A- 0 127 783**
**EP-A- 0 136 280**
**DE-A- 2 930 546**
**US-A- 4 119 660**
**US-A- 4 473 507**

(73) Patentinhaber: **Degussa Aktiengesellschaft, Weissfrauenstrasse 9, D-6000 Frankfurt am Main 1 (DE)**
Patentinhaber: **Henkel Kommanditgesellschaft auf Aktien, Postfach 1100 Henkelstrasse 67, D-4000 Düsseldorf-Holthausen (DE)**

(72) Erfinder: **Dankowski, Manfred, Dr., Stifterstrasse 14, D-8757 Karlstein (DE)**

**EP 0 179 223 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von stabilisierten, substituierten Diperoxybernsteinsäuren. Derartige Verbindungen werden bei der Wäsche oder Bleiche von Textilien eingesetzt, da ihre Wirkung schon bei Temperaturen unterhalb von 80 °C eintritt.

Es ist bereits eine Reihe verschiedener Verfahren zur Herstellung von Peroxycarbonsäure veröffentlicht worden.

Die EP-A2 0 045 290 betrifft ein Verfahren zur Herstellung von Peroxycarbonsäuren, bei dem die Ausgangscarbonsäuren in konzentrierter Schwefelsäure gelöst und die durch Oxidation mit Wasserstoffperoxid gebildeten Persäuren kontinuierlich mit einem organischen Lösungsmittel extrahiert werden.

In der US-PS 4 119 660 wird ebenfalls ein Verfahren beschrieben, bei dem die Ausgangscarbonsäuren in grossen Überschüssen von Schwefelsäure gelöst und anschliessend zu Persäuren umgesetzt werden.

Die EP-A 10 083 560 betrifft die Herstellung von substituierten Diperoxybernsteinsäuren durch die Umsetzung der entsprechenden, in Methansulfonsäure gelösten Anhydride mit Wasserstoffperoxid.

Aus der EP-A 0 136 280 ist ein Verfahren zur Herstellung von substituierten Diperoxybernsteinsäuren durch Umsetzung der substituierten Bernsteinsäureanhydride in einer Mischung aus konzentrierter Schwefelsäure und Wasserstoffperoxid bekannt. Das Molverhältnis Schwefelsäure zu Anhydrid beträgt hierbei 10–60:1, vorzugsweise 20–40:1. Die Schwefelsäure dient offensichtlich als Lösungsmittel für das eingesetzte Anhydrid.

Gemäss dem Verfahren der DE-OS 2 930 546 werden wasserunlösliche aliphatische oder aromatische Peroxycarbonsäuren nach der Reaktion der Carbonsäure mit Wasserstoffperoxid in Gegenwart von Schwefelsäure phlegmatisiert, indem ein Alkalihydroxid zugesetzt und dabei ein pH-Wert von 2–6 eingestellt wird. Phlegmatisierungsmittel ist somit in situ gebildetes Alkalisulfat. Die Phlegmatisierung erfolgt bei 0 bis 30 °C, d.h. unterhalb des Umwandlungspunktes, von Natriumsulfat-dekahydrat in wasserfreies Natriumsulfat, woraus technische Probleme resultieren.

Aufgabe der Erfindung ist ein Verfahren zur Herstellung stabilisierter substituierter Diperoxybernsteinsäuren, das ohne organische Lösungsmittel auskommt und bei dem die Ausgangssubstanzen nicht in grossen Mengen überschüssiger Schwefelsäure gelöst werden müssen.

Gegenstand der Erfindung ist ein Verfahren zur Herstellung von stabilisierten substituierten Diperoxybernsteinsäuren durch Umsetzung eines Anhydrids der Formel

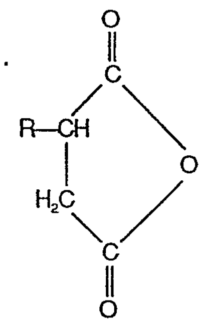

in der R einen linearen oder verzweigten Alkylrest mit 4 bis 20 C-Atomen oder Aryl bedeutet, mit einer Oxidationsmischung aus Wasserstoffperoxid, Wasser und Schwefelsäure unter Einsatz eines Molverhältnisses von Wasserstoffperoxid zu Anhydrid von 2 bis 20 zu 1 bei 20–90 °C, das dadurch gekennzeichnet ist, dass man zur Umsetzung Schwefelsäure und Anhydrid im Molverhältnis von 2 bis 10 zu 1 einsetzt und das Anhydrid in der Oxidationsmischung suspendiert und die Stabilisierung unter Verwendung von in situ gebildetem Natriumsulfat als Phlegmatisierungsmittel vornimmt, indem man durch Zugabe von Natronlauge oder Natriumcarbonatlösung bei Temperaturen oberhalb 32,4 °C einen pH-Wert von 2 bis 6 einstellt und dabei mindestens einen Teil der im Reaktionsgemisch vorhandenen Schwefelsäure in Natriumsulfat überführt, und das Reaktionsprodukt in bekannter Weise aufarbeitet.

Man führt die Umsetzung mit einem Molverhältnis von Wasserstoffperoxid zu Anhydrid von 2 bis 20 zu 1, bevorzugt 6 bis 12 zu 1, aus.

Bevorzugt werden Anhydride von alkylierten Bernsteinsäuren. Besonders geeignet sind 2-Octylbernsteinsäureanhydrid, 2-Decylbernsteinsäureanhydrid, 2-Dodecylbernsteinsäureanhydrid, 2-Tetradecylbernsteinsäureanhydrid.

Bei der Durchführung des erfindungsgemässen Verfahrens erweist es sich als günstig, Schwefelsäure und Anhydrid im Molverhältnis 2 bis 10 zu 1, bevorzugt 6 bis 10 zu 1, einzusetzen. Für die Auswahl der Molverhältnisse allgemein ist wesentlich, dass das Anhydrid mit der jeweiligen Oxidationsmischung eine Suspension bildet.

Die Umsetzung wird bei Temperaturen zwischen 20–90 °C, bevorzugt bei 50–70 °C, durchgeführt. Wasserstoffperoxid wird in Konzentrationen von 20 bis 99 Gew.-%, bevorzugt 40 bis 60 Gew.-%, eingesetzt; Schwefelsäure in Konzentrationen von 20 bis 98 Gew.-%, bevorzugt 90 bis 98 Gew.-%.

Die Peroxycarbonsäuren lassen sich hervorragend stabilisieren, wenn man nach der Umsetzung auf dem suspendierten Produkt vor dessen Abtrennen aus dem Reaktionsgemisch Natriumsulfat als Phlegmatisierungsmittel in situ erzeugt.

Besonders geeignet ist hierfür ein Verfahren, bei dem man die Umsetzung der Anhydride mit der Oxidationsmischung bei 50–70 °C vornimmt, darauf das Reaktionsgemisch auf Temperaturen von 45 °C–32 °C abkühlt, eine wässrige Alkalisulfatlösung zusetzt, den pH-Wert der Mischung auf 2-6, bevorzugt 3 bis 4, mit Hilfe von Natronlauge oder Natriumcarbonatlösung einstellt und danach das Reaktionsprodukt in bekannter Weise aufarbeitet. Auch eine Teilneutralisation der Mineralsäure ist möglich. Natirumhydroxid oder Natriumcarbonat werden als wässrige Lösungen mit 5-50 Gew.-% eingesetzt. Bevorzugt setzt man 30-50 gew.-%ige Natronlauge ein.

Durch die in situ-Bildung von Natriumsulfat aus der im Reaktionsgemisch vorhandenen Schwefelsäure und zugesetzter Natronlauge bei Temperaturen oberhalb des Umwandlungspunktes von Natriumsulfat-Dekahydrat zu wasserfreiem Natriumsulfat, d.h. bei Temperaturen oberhalb 32,4°C, wird der Restfeuchtegehalt der Peroxycarbonsäuren nach dem Zentrifugieren um bis zu 50% vermindert.

Zusätzlich zu in situ gebildetem Natriumsulfat kann Natriumsulfat auch fest und/oder als wässrige Lösung zugesetzt werden.

Das Phlegmatisierungsmittel soll bevorzugt in Mengen von 0,1 bis 90 Gew.-%, bezogen auf das fertige Produkt, anwesend sein.

Die Lagerstabilität wird durch das Phlegmatisierungsmittel in starker Weise erhöht.

## Patentansprüche

1. Verfahren zur Herstellung von stabilisierten substituierten Diperoxybernsteinsäuren durch Umsetzung eines Anhydrids der Formel

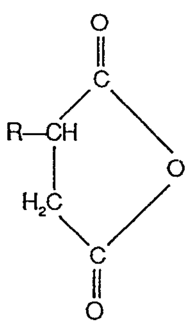

in der R einen linearen oder verzweigten Alkylrest mit 4 bis 20 C-Atomen oder Aryl bedeutet, mit einer Oxidationsmischung aus Wasserstoffperoxid, Wasser und Schwefelsäure unter Einsatz eines Molverhältnisses von Wasserstoffperoxid zu Anhydrid von 2 bis 20 zu 1 bei 20-90°C, dadurch gekennzeichnet, dass man zur Umsetzung Schwefelsäure und Anhydrid im Molverhältnis von 2 bis 10 zu 1 einsetzt und das Anhydrid in der Oxidationsmischung suspendiert und die Stabilisierung unter Verwendung von in situ gebildetem Natriumsulfat als Phlegmatisierungsmittel vornimmt, indem man durch Zugabe von Natronlauge oder Natriumcarbonatlösung bei Temperaturen oberhalb 32,4°C einen pH-Wert von 2 bis 6 einstellt und dabei mindestens einen Teil der im Reaktionsgemisch vorhandenen Schwefelsäure in Natriumsulfat überführt, und das Reaktionsprodukt in bekannter Weise aufarbeitet.

2. Verfahren nach Anspruch 1, dadurch gekennzeichnet, dass man bei der in situ-Bildung von Natriumsulfat einen pH-Wert von 3 bis 4 einstellt.

3. Verfahren nach Anspruch 1 oder 2, dadurch gekennzeichnet, dass man die Umsetzung bei Temperaturen von 50°C bis 70°C vornimmt, darauf das Reaktionsgemisch auf 45°C bis 32°C abkühlt und eine wässrige Alkalisulfatlösung zusetzt, worauf man die in situ-Bildung von Natriumsulfat vornimmt.

## Claims

1. Process for the preparation of stabilised, substituted diperoxysuccinic acids by reacting an anhydride of the formula

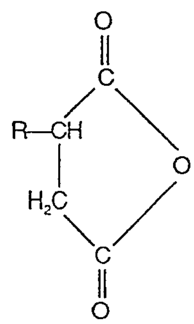

in which R denotes a linear or branched alkyl radical having 4 to 20 C atoms or aryl, with an oxidation mixture consisting of hydrogen peroxide, water and sulphuric acid, using a molar ratio of hydrogen peroxide to anhydride of 2 up to 20 to 1, at 20-90°C, characterized in that sulphuric acid and the anhydride are employed for the reaction in a molar ratio of 2 up to 10 to 1, and the anhydride is suspended in the oxidation mixture and the stabilisation is effected using sodium sulphate formed in situ as a stabilising agent, by adjusting the pH to a value of 2 to 6 by adding sodium hydroxide or sodium carbonate solution at temperatures above 32.4°C, and thus converting at least a fraction of the sulphuric acid present in the reaction mixture into sodium sulphate, and working up the reaction product in a known manner.

2. Process according to Claim 1, characterized in that the pH is adjusted to a value of 3 to 4 in the formation of sodium sulphate in situ.

3. Process according to Claim 1 or 2, characterized in that the reaction is carried out at temperatures from 50°C to 70°C, the reaction mixture is then cooled to 45°C to 32°C and an aqueous solution of alkali metal sulphate is added, after which the formation of sodium sulphate in situ is carried out.

## Revendications

1. Procédé pour la préparation d'acides diperoxysucciniques substitués stabilisés, par réaction d'un anhydride de formule

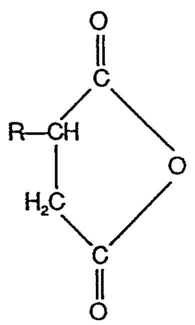

dans laquelle R représente un radical alkyle linéaire ou ramifié ayant de 4 à 20 atomes de carbone, ou un radical aryle, avec un mélange d'oxydation à base de peroxyde d'hydrogène, eau et acide sulfurique, avec utilisation d'un rapport molaire du peroxyde d'hydrogène à l'anhydride allant de 2:1 à 20:1, à 20-90°C, procédé caractérisé en ce que, dans la réaction, on utilise l'acide sulfurique et l'anhydride dans un rapport molaire allant de 2:1 à 10:1 et on met en suspension l'anhydride dans le mélange d'oxydation et on effectue la stabilisation avec utilisation, en tant qu'agent de flegmatisation, du sulfate de sodium formé in situ, en ajustant le pH à une valeur de 2-6 par addition d'une solution d'hydroxyde de sodium ou d'une solution de carbonate de sodium, à dès températures supérieures à 32,4°C, et au moins une partie de l'acide sulfurique présent dans le mélange réactionnel est ainsi convertie en sulfate de sodium, et on achève de façon connue le traitement du produit de réaction.

2. Procédé selon la revendication 1, caractérisé en ce que, lors de la formation du sulfate de sodium in situ, on ajuste le pH à une valeur de 3 à 4.

3. Procédé selon la revendication 1 ou 2, caractérisé en ce que l'on effectue la réaction à une température de 50°C à 70°C, puis on refroidit le mélange réactionnel jusqu'à une température de 45°C à 32°C, et on ajoute une solution aqueuse d'un sulfate alcalin, à la suite de quoi on engendre in situ la formation de sulfate de sodium.